Europäisches Patentamt

European Patent Office

Office européen des brevets

(11) Publication number : **0 527 606 A2**

## (12) EUROPEAN PATENT APPLICATION

(21) Application number : **92307229.2**

(22) Date of filing : **07.08.92**

(51) Int. Cl.[5] : **G01N 33/15**

(30) Priority : **14.08.91 US 746016**

(43) Date of publication of application :
**17.02.93 Bulletin 93/07**

(84) Designated Contracting States :
**AT BE CH DE ES FR GB IT LI NL SE**

(71) Applicant : **ROHM AND HAAS COMPANY
Independence Mall West
Philadelphia Pennsylvania 19105 (US)**

(72) Inventor : **Miller, Muriel Elizabeth
509 E.Montgomery Avenue
North Wales, Pennsylvania 19454 (US)**
Inventor : **Maikner, John Joseph
2455 Steinsburg Road
Quakertown, Pennsylvania 18951 (US)**

(74) Representative : **Tanner, James Percival et al
ROHM AND HAAS (UK) LTD. European
Operations Patent Department Lennig House
2 Mason's Avenue
Croydon CR9 3NB (GB)**

(54) **Apparatus and process for measuring release rates of bound solutes from binding compositions.**

(57) The present invention provides an apparatus, and a method utilizing said apparatus, for determining the release rate of bound solutes from finely divided compositions to which they are bound. The apparatus comprises : a non-porous vessel having walls, an inlet and an outlet ; a first and second porous support disposed within, and sealed to the walls of, the vessel ; an enclosed volume for housing the finely divided composition having the solute bound thereto, the enclosed volume being bounded by the first and second porous supports and the walls of the vessel ; and a collecting region disposed within the vessel between the second support and the outlet, the collecting region having a volume which is smaller than about 50% of the enclosed volume.

FIG.1.

EP 0 527 606 A2

The present invention is concerned with an apparatus and a method for determining the release rate of bound solutes from compositions in which they are bound. For example, the present invention provides an apparatus and a method for determining the rate of release of an adsorbed solute from a finely divided adsorbent material upon which it is adsorbed.

In evaluating drug-delivery systems it is helpful to estimate the rate at which a drug is released into the organism being treated. Slow-release coatings, time-release capsules and the like have been evaluated by immersing the drug-containing pill, capsule or the like in a liquid that simulates the physiological fluid to which it will be exposed in use, and measuring the increasing concentration of the drug in the liquid.

Standard apparatus for release-rate determinations are specified by the United States Pharmacopeia, British Pharmacopeia, U.S. National Formulary and similar organizations in other countries. In general, the apparatus specified is intended for determining the dissolution rate of a pill or tablet, and is not well suited for determinations involving finely divided materials.

Ion-exchange resins and adsorbent resins have been used to control the release rates of drugs into the stomach and intestines, onto the skin, and the like. The drug is adsorbed or exchanged onto the resin, and then is desorbed, or eluted, into the fluids of the body. The size of these resins, in bead or powder form, is typically from about 50 mesh to smaller than 400 mesh (300 - 25 micrometers ($\mu$m)), and retaining them in an apparatus designed for holding a tablet with dimensions of several millimeters has proved difficult. One approach has been to wrap the resin in a fine-mesh fabric, creating an article reminiscent of a tea bag, which is then tested in the same manner as a tablet. An objection to applying this test to finely divided resins is that the finer powders are difficult to retain in the fabric. Another objection is that the eluent liquid must diffuse throughout the volume of the powder mass, and the time required for this diffusion degrades the accuracy of kinetic measurements such as release rate.

Yet another problem in measuring drug release rates of small samples relates to "dead volume", the volume of the apparatus which contains the liquid that has already been exposed to the drug carrier, but whose drug concentration has not yet been measured. Large dead volumes with respect to the sample volume permit mixing of liquid exposed to the drug earlier with liquid more recently exposed, so that instantaneous concentration of the drug in the liquid may not be measured accurately and the sensitivity of the release-rate assay is thus degraded.

Thus, an object of the present invention is to provide an apparatus and method broadly applicable to determining, at high sensitivity, the release rate of adsorbed, exchanged or entrained materials from finely divided adsorbents, ion exchangers or entraining compositions. Other objects of the present invention will be apparent from the following description and Examples.

According to the present invention there is provided an apparatus for determining release rates of bound solutes, e.g. adsorbed materials, from finely divided compositions, e.g. adsorbents or ion-exchange resins, to which the solutes are bound, which comprises:-

(a) a non-porous vessel having walls, an inlet and an outlet;

(b) a first and second porous support disposed parallel to one another within, and sealed to the walls of, the vessel, the supports having pores smaller than about 50 $\mu$m;

(c) an enclosed volume bounded by the first and second supports and the wall of the vessel; and

(d) a collecting region disposed within the vessel between the second support and the outlet, the collecting region having a volume which is smaller than about 50% of the enclosed volume, and is preferably smaller than about 500 microliters ($\mu$l), the vessel having a means for introducing, into the enclosed volume, a finely divided composition capable of binding a solute.

According to the present invention there is also provided a method for determining the release rate of bound solutes from finely divided compositions to which they are bound, which comprises the steps of:-

(a) disposing the finely divided composition within the enclose volume of an apparatus according to the present invention, and wherein the solute is bound to the finely divided composition prior to, or subsequent to, the disposition of the finely divided composition within the enclosed volume of the apparatus;

(b) introducing an eluting liquid into the inlet of the non-porous vessel of the apparatus so that it passes through the finely divided composition, and flows from the outlet of the vessel; and

(c) determining the concentration of the solute in the eluting liquid that flows from the outlet of the vessel.

In a preferred embodiment of the present invention there is provided a method for determining release rates of bound solutes from finely divided compositions to which they are bound, which comprises:-

(a) disposing the finely divided composition, to which is bound the solute, within a non-porous vessel having

(i) walls, an inlet and an outlet,

(ii) a first and second porous support disposed parallel to one another within, and sealed to the walls of, the vessel, the supports having pores smaller than about 50 $\mu$m,

(iii) an enclosed volume bounded by the first and second supports and the wall of the vessel, and

(iv) a collecting region disposed within the vessel between the second support and the outlet, the collecting region having a volume which is smaller than about 50% of the enclosed volume, and is preferably smaller than about 500 microliters ($\mu$l), the vessel having a means for introducing a finely divided adsorbent or ion-exchange resin into the enclosed volume;

(b) introducing an eluting liquid into the inlet of the vessel so that it passes through the adsorbent or ion-exchange material, and flows from the outlet of the vessel; and

(c) determining the concentration of the solute in the eluting liquid that flows from the outlet of the vessel.

In the following description reference is often made, for convenience, to the embodiment in which the binding of the solute to the finely divided composition is by way of adsorption. However, it is to be understood that such description is generally applicable to all types of binding contemplated within the present invention.

Release-rate determination is a kinetic measurement, and successful determinations of release rate should allow close approximation of the actual, instantaneous concentration of released, bindable material, e.g. desorbed, adsorbable material, in the eluent liquid. The apparatus of the present invention encourages such successful release-rate determinations.

The apparatus of the present invention, and particularly the walls of the apparatus, may be made from glass, silica, metal or polymeric material. Whatever material is used, it should be selected for resistance to attack by the liquids employed in the release-rate determination, for example those used in loading or eluting the adsorbable material or cleaning the system. It should also resist whatever pressures are required by the particular determination to force liquid through the system. Stainless steel and similar non-reactive metals are examples of metals from which such a selection may be made, as are more reactive metals plated with non-reactive metals. Polyethylene, polypropylene, poly(tetrafluoroethylene), polycarbonate and the like are examples of polymeric materials from which such a selection may be made. Other suitable materials include polymer-coated metals and the like, in which the polymer provides the lack of reactivity and the metal provides the pressure resistance and strength.

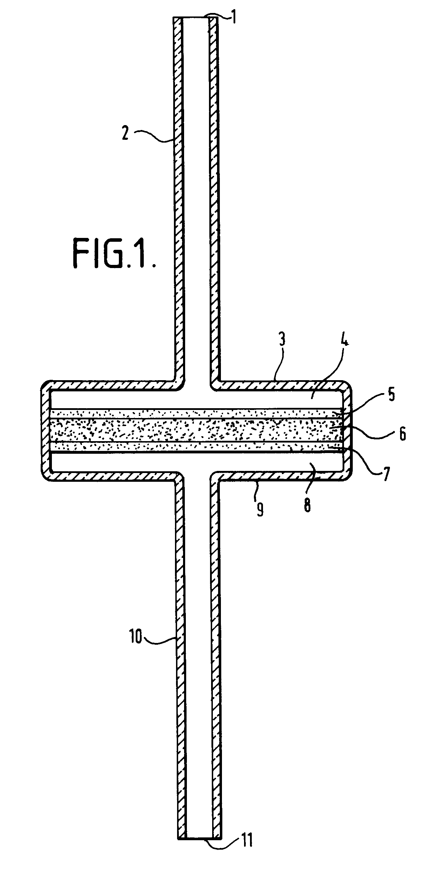

The present invention will now be further described with reference to the accompanying drawing, which is depicted as Figure 1 and shows an embodiment of the apparatus of the present invention. Referring to Figure 1, the apparatus comprises an inlet opening (1) of the apparatus; a wall (2) forming an inlet tube, which leads to an inlet dispersing volume (4) defined by an inlet enclosing wall (3); a first support (5); an enclosed volume (6); a second support (7); a collecting volume (8) defined by an outlet enclosing wall (9); and a tube (10) leading to an outlet opening (11).

The supports (e.g. supports 5 and 7 in the embodiment illustrated in Figure 1) may be screens, for example metal screens such as fine-mesh screens of stainless steel, or they may be sintered, porous materials such as sintered or fritted glass or metals, or they may be screens, sintered plates or porous polymers including polymer films, for example porous poly(tetrafluoroethylene). The materials from which the supports are made should be selected according to criteria of chemical and pressure resistance similar to those for the apparatus walls. The supports should be affixed to the walls of the apparatus in such a manner that liquid cannot leak past the support edges. The porosity of the supports, that is, the effective size of the largest pores, should be fine enough to retain essentially all of the adsorbing material, and is thus selected based upon the minimum particle size of the adsorbing material. The porosity of the supports is preferably smaller than 50 $\mu$m, more preferably smaller than 25 $\mu$m. Because pressure drop across the supports increases as the porosity decreases, the strength of the support must also be considered, and particularly small porosities may require composite materials, for example a porous polymer further supported by a metal screen, or other special materials or configurations which will be apparent to those skilled in the art, to provide the requisite combination of porosity and strength.

A means is provided for sealing a sample of the adsorbing material within the enclosed volume (e.g. the enclosed volume 6 in the embodiment illustrated in Figure 1) between the supports. In an embodiment which exemplifies such a means, the apparatus is formed in a pair of separable parts which, in use, are joined between the two supports to seal the enclosed volume (e.g. the enclosed volume 6 in the embodiment illustrated in Figure 1). The parts may be joined by, for example, mating screw threads on the two parts, or by clamping means which may be integral or external to the parts of the apparatus. Other sealing means will be apparent to those skilled in the art.

The finely divided composition (also referred to herein as the adsorbing material) to which the solute is bound, and which is useful in the practice of the present invention, is a composition which will bind, ionically, by Van der Waals force, or by other reversible binding mechanisms, the solute, and is exemplified by, but not limited to, adsorbent resins, e.g. inert, macroporous, polymeric adsorbents which will passively adsorb the solute, i.e. adsorbable drug or other material, into their macropores, ion-exchange resins which will actively exchange ions from the solute for ionic sites on the finely divided composition, and chelating resins which will bind a particular dissolved material to active sites on the resin. One skilled in the art will readily understand related finely divided compositions which will bind the solute, and which may also be referred to herein as adsorbing materials, and the employment of them in practising the process of the present invention.

The finely divided composition may, for example, have a particle size of from 25μm to 700μm, preferably from 100μm to 500 μm.

The process by which the solute (also referred to herein as the adsorbable material) is bound, or loaded, onto the adsorbing material will similarly be apparent to those skilled in the art, and generally involves contact between the adsorbing material and the adsorbable material. As an example, the solute may be bound to the finely divided composition prior to its being disposed within the vessel, for example, a suspension of the adsorbing material in liquid may contact a solution, suspension or dispersion of the adsorbable material in the same liquid or in another liquid which may be mixed with that liquid suspending the adsorbing material. As another example, the adsorbing material may be disposed within the enclosed volume (e.g. the enclosed volume 6 in the embodiment illustrated in Figure 1) as described below for the release-rate determination, and the liquid containing the adsorbable material may be passed through the apparatus. The efficiency of the loading may be increased by recycling the solution, suspension or dispersion of the adsorbable material from the outlet back into the inlet. Although non-aqueous liquids may be used, the preferred liquid for suspending the adsorbing material, and for dissolving, suspending or dispersing the adsorbable material, is water, which may be mixed with other liquids or may contain dissolved salts or other materials, or suspended or dispersed materials.

The adsorbable material may be a drug, or it may be another material which may be adsorbed onto the adsorbing material as described above. In either case, the rate at which the adsorbed material is released from the adsorbing material may be determined according to the process of the present invention.

Preferably, the amount of adsorbable material loaded onto the adsorbing material should be known. Methods which are readily apparent to those skilled in the art may be used to determine the amount of material adsorbed, as for instance, by measuring the concentration of adsorbable material in a known volume of solvent both before and after contact with the adsorbing material, and calculating the total loss of the absorbable material from the difference in these concentrations.

In practising the process of the present invention the adsorbing material upon which the adsorbable drug or other material is adsorbed is placed in the enclosed volume of the apparatus so that it forms a thin layer. An eluent liquid, which may be an actual physiological fluid to which the finely divided composition, e.g. the adsorbent or ion-exchange material, will be exposed or a simulant for such a fluid, is introduced into the inlet opening under pressure, flows through the material in the enclosed volume, and exits through the collecting region and thence through the outlet opening. The eluent liquid elutes the adsorbable material from the adsorbing material into the liquid and transports it through the second support, through the minimized collecting volume and out of the outlet. The eluent liquid may be introduced into the inlet opening under the pressure provided by gravity, or more preferably under the pressure provided by pumping it from a liquid reservoir.

A detector, for example one which measures the absorption of ultraviolet-light by the liquid, is preferably placed downstream from the outlet opening, for example between the outlet opening and the eluent reservoir in the case where the eluent is recycled back to the eluent reservoir. Alternatively, samples may be taken from the liquid exiting from the outlet opening, and the concentration of eluate determined in these samples. Such samples may be taken continuously, as with a stream splitter which diverts a portion of the exiting liquid, or discontinuously as with a pipette. The samples may be taken automatically, as with the stream splitter described above or with an automatic pipetting apparatus, or they may be taken manually. Various colorimetric or photometric determinations of eluate concentration, or other analytical determinations appropriate to the particular eluate, may be selected by one skilled in the art. Preferably, the concentration of the solute in the eluting liquid flowing from the outlet of the vessel is determined spectrophotometrically.

In a preferred embodiment of the present invention the concentration of the solute flowing from the outlet of the vessel is determined and recorded continuously.

The accuracy of the measurement of the concentration of the solute in the eluting liquid that flows from the outlet of the vessel may be significantly improved where the volume through which the eluting liquid travels from the second porous support to the detector is minimized.

In one embodiment of the present invention, the rate at which adsorbed materials are released from a small sample of a finely divided adsorbing material, such as a polymeric adsorbent or ion exchange resin, may be determined by confining the sample in a broad, thin configuration between a pair of porous supports, passing an eluting liquid through the porous supports and the sample to elute the adsorbed material, and detecting the amount of adsorbed material which has eluted into the eluting liquid.

The apparatus may have additional, optional features not recited in the discussion above, for example, connections, such as tubes, through which the eluting liquid may flow from the inlet to the first support, and from the collecting region to the outlet, and further including a distributing volume between the inlet tube and the first support, defined by the wall of the apparatus between the inlet or any such connection as described above and the first support, into which the eluting liquid may flow and cover the surface of the first support. Other additional features which may facilitate the process of the present invention will be apparent to those skilled in the art.

EXAMPLES

The following Examples are presented to illustrate preferred embodiments of the present invention. All proportions and percentages are by weight unless otherwise stated, and all reagents used are of good commercial quality unless otherwise stated.

Example 1

This Example illustrates determination of the release rate of dextromethorphan from a cation-exchange resin using the apparatus of the present invention, and that the effect of a parameter such as temperature upon release rate may readily be measured using the process of the present invention.

The apparatus of the present invention used in this Example had supports of 25-μm porosity and 25-milliiseter (mm) diameter, enclosing a 970-μl sample volume. The volume above the inlet support was 360 μl, and the volume of the collection region below the outlet support was 370 μl. Dextromethorphan was loaded onto a styrene-based, strong-acid, cation exchange resin having a particle size range of 100-500 μm and a cation exchange capacity of 1.9 milliequivalents per milliliter (meq/ml, wet basis) by mixing 3.6 g resin with 60 ml of a 10 milligram/milliliter (mg/ml) stock aqueous dextromethorphan hydrobromide solution (for a total of 600 mg dextromethorphan hydrobromide) and shaking the mixture at room temperature for at least four hours. Uptake of the dextromethorphan was measured by removing a filtered, approximately 1-ml sample from the mixture and measuring its ultraviolet absorbance at 275 nanometers (nm). The dextromethorphan level in the solution was determined by comparing this to the absorbance of standard solutions, and the dextromethorphan loss from the solution was calculated as the amount of dextromethorphan loaded onto the resin. Approximately 97% of the available dextromethorphan (approximately 0.162 mg dextromethorphan per mg resin or 0.14 mg dextromethorphan per mg of the dextromethorphan-resin complex, or resinate) was loaded onto the resin within four hours. Samples (400 mg) of this resinate were placed between the supports of the apparatus, and the dextromethorphan was eluted from the resinate with a 0.9% (weight/volume) aqueous sodium chloride solution flowing at a constant rate of 3 ml/minute. The sodium chloride solution and the apparatus were maintained at a constant temperature throughout each elution, the respective temperatures being shown in Table I. The concentration of dextromethorphan in the eluting liquid was measured spectrophotometrically at 275 nm, and the results for each sample, with specific elution conditions, are shown below in Table I.

Example 2

This comparative Example illustrates the use of an apparatus having a relatively large collection region for determining the elution of dextromethorphan from the dextromethorphan resinate described in Example 1.

The apparatus used in this Example had porous supports of 25-μm porosity and 42-mm diameter enclosing a sample volume of 6.1 ml, and had a collection region of 3.2 ml. This apparatus was of commercial manufacture, the Swinnex filter apparatus from Millipore Corporation, Bedford, MA 01730. 400-mg samples of the dextromethorphan resinate were similarly placed between the porous supports, and the dextromethorphan was eluted by 0.9% (weight/volume) aqueous sodium chloride solution flowing at a constant rate of 3 ml/minute. The concentration of dextromethorphan in the eluting liquid was measured spectrophotometrically as 275 nm, and the results and elution temperatures are shown below in Table I.

## Table I

### Release of Dextromethorphan with Time

| Time (minutes) | Example 1 Present Invention | | Example 2 Comparative Example | |
|---|---|---|---|---|
| | 25°C | 37°C | 25°C | 37°C |
| 2 | 3.9 | 3.0 | — — | — — |
| 3 | — — | — — | 1.0 | 1.1 |
| 10 | 6.7 | 7.7 | 5.1 | 4.9 |
| 20 | 10.0 | 12.9 | 8.3 | 8.7 |
| 40 | 16.3 | 21.3 | 13.8 | 14.7 |
| 60 | 21.5 | 28.1 | 18.6 | 19.7 |
| 80 | 25.9 | 34.5 | 22.3 | 24.0 |
| 100 | 29.9 | 39.3 | 25.9 | 27.4 |
| 120 | 33.9 | 43.5 | 28.7 | 30.4 |
| 160 | 36.4 | 45.2 | 31.5 | 33.6 |
| 180 | 39.1 | 49.1 | 37.3 | 36.4 |

As may be seen from Table I, the rate of dextromethorphan release measured at room temperature is significantly different for the apparatus of the present invention than for the sample holder having the larger sample volume and collection region, and this measured difference in release rate is increased at higher temperatures. The apparatus of the present invention thus retains sufficient sensitivity to rate-related differences in concentration to permit detection of this difference in release rate with temperature, while the comparative apparatus is unable to distinguish the rate difference.

"Swinnex" is a trademark which may be registered in one or more of the designated countries.

## Claims

1. An apparatus for determining the release rate of bound solutes from finely divided compositions to which they are bound, which comprises
(a) a non-porous vessel having walls, an inlet and an outlet,
(b) a first and second porous support disposed parallel to one another within, and sealed to the walls of, the vessel, the supports having pores smaller than about 50 μm,
(c) an enclosed volume bounded by the first and second supports and the wall of the vessel,
d) a collecting region disposed within the vessel between the second support and the outlet, the collecting region having a volume which is smaller than about 50% of the enclosed volume, the vessel having a means for introducing, into the enclosed volume, a finely divided composition capable of binding a solute.

2. An apparatus as claimed in Claim 1, wherein the collecting region is smaller than about 500 microliters (μl).

3. An apparatus as claimed in Claim 1 or 2, wherein the walls are metal or glass.

4. An apparatus of as claimed in any preceding claim, wherein the first support, second support or both is/are: porous, fritted glass; porous, fritted metal; metal screens; or porous polymer.

5. A method for determining the release rate of bound solutes from finely divided compositions to which they are bound, which comprises the steps of:
(a) disposing the finely divided composition within the enclosed volume of an apparatus as claimed in any preceding claim, and wherein the solute is bound to the finely divided composition prior to, or subsequent to, the disposition of the finely divided composition within the enclosed volume of the apparatus;

(b) introducing an eluting liquid into the inlet of the non-porous vessel of the apparatus so that it passes through the finely divided composition, and flows from the outlet of the vessel; and

(c) determining the concentration of the solute in the eluting liquid that flows from the outlet of the vessel.

6. A method as claimed in Claim 5, wherein a step of binding the solute to the finely divided composition is carried out prior to its being disposed within the vessel.

7. A method as claimed in Claim 5 or Claim 6, wherein the solute is a drug.

8. A method as claimed in any of Claims 5 to 7, wherein the concentration of the solute in the eluting liquid flowing from the outlet of the vessel is determined spectrophotometrically.

9. A method as claimed in any of Claims 5 to 8, wherein the concentration of the solute in the eluting liquid flowing from the outlet of the vessel is determined and recorded continuously.

10. A method as claimed in any of Claims 5 to 7, wherein the concentration of the solute in the eluting liquid flowing from the outlet of the vessel is determined by periodically withdrawing samples from said eluting liquid flowing from the outlet of the vessel.

11. A method as claimed in any of claims 5 to 10, wherein the finely divided composition is a finely divided absorbent or ion exchange resin.

FIG.1.